# EUROPEAN PATENT APPLICATION

(11) **EP 4 477 737 A1**
(43) Date of publication of application: **18.12.2024**
(21) Application number: 24020121.0
(22) Date of filing: 16.04.2024
(51) Int. Cl.: C12N 1/14, C12P 19/32, C12R 1/885

(54) **THE METHOD OF OBTAINING AND EXTRACTION OF SECONDARY FUNGAL METABOLITES**

(30) Priority: 17.04.2023 PL 44443723
(71) Applicant: Uniwersytet Mikolaja Kopernika w Toruniu, 87-100 Torun (PL)
(72) Inventor: Dabrowska, Grazyna Barbara, 87-100 Torun (PL); Kulasek, Milena, 87-100 Torun (PL); Mierek-Adamska, Agnieszka, 87-100 Torun (PL); Turkan, Sena, 87-100 Torun (PL)

(57) **Abstract**

The subject of the invention is the method of obtaining and extraction of secondary fungal metabolites characterized in that the *T. Viride* mycelium in the amount 75-100 mg is shredded and nucleotides are separated in such a manner that the mycelium is suspended in 1.5-2 N formic acid and incubated on ice preferably for 15 to 30 min, then centrifuged and the supernatant is collected, and then ammonium acetate NH4OAc, the 13C ppGpp internal standard are added and introduced into an already prepared SPE column by rinsing with methanol MeOH and ammonium acetate in the amount from 35 to 60 mM, and then the column with the introduced sample is rinsed with ammonium acetate NH4OAc in the amount from 30 to 70 mM preferably 50 mM at the pH from 4 to 5 preferably pH 4.5 and methanol MeOH and nucleotides are eluted with a MeOH/ H2O/NH4OH solution, and the thus prepared eluate is lyophilized, dissolved in water and filtered in columns for DNA purification. Preferably the mycelium is shredded by grinding in a mortar preferably at the temperature from 20 to 25°C.

## Description

The subject of the invention is a method of obtaining and extraction of secondary fungal metabolites for agricultural applications.

Due to their inability to escape from inimical environmental conditions, plants had to develop efficient mechanisms that would allow them to adjust their growth and development to the already existing conditions as well as to efficiently defend themselves against stress. Stringent response is one of the key elements combining these processes, and its key effector is guanosine tetraphosphate (denoted as ppGpp, alarm one), a hyperphosphorylated nucleotide present in bacteria (Cashel, 1969), plant chloroplasts (Romand et al., 2022), algae (Avilan et al., 2021) as well as animals and humans (Ito et al., 2020). In the case of fungi, these atypical alarmones have not been identified yet. On the molecular level, ppGpp regulates numerous cell functions involved, among others, in the DNA replication (Levine et al., 1991; Wang et al., 2007), transcription, translation (Srivatsan & Wang, 2008), thus enabling the adjustment to changes in environmental conditions (drought, freezes, salination, and others). As suggested in the latest reports, the activation of the stringent response in plants occurs in chloroplasts, which in the cascade way influences a plant cell metabolism as well as plastid and nuclear genes expression (Ito et al., 2022, Field 2018). In plants and algae, the accumulation of ppGpp can inhibit the chloroplast genes expression and influence photosynthesis, nutrient remobilization, growth, and immunity (Boniecka et al., 2018). Additionally, ppGpp biosynthesis is fundamental to the acclimatization to nitrogen deficit in *Arabidopsis arenosa* which takes place via the rearrangement of the photosynthetic electron transport chain and coupling of chloroplast and nuclear genes expression (Romand et al., 2022).

An uncontrollably high amount of ppGpp in plant cells results in a significant decrease in photosynthesis parameters connected with the destruction of photosynthetic systems (Romand et al., 2022), which can cause dwarfism in plants (Ihara & Masuda, 2016). The compound could be an efficient, highly degradable herbicide. On the other hand, since ppGpp participates in the acclimatization of plants to different kinds of stress, spraying them with its solution at an appropriate concentration will be a form of a biodegradable "vaccine" preparing cultivated plants for suddenly appearing adverse environmental conditions, which will enable higher harvest predictability.

In plants, the stringent response is regulated by synthetases and hydrolases of alarmones coded by *RSH* genes (RelA/SpoT homolog) and it is initiated through the accumulation of alarmones in response to the activity of stress factors (D 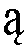 browska et al., 2006) resulting from: injuring, the presence of microorganisms (Sierakowska et al., 2019), viruses (Abdelkefi et al., 2018), salination (D 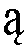 browska et al., 2021), treatment with abscisic acid (ABA), changes in lighting conditions (Prusińka et al., 2019), drought stress and oxidative stress (Prusińska et al., 2019). Under physiological conditions, these proteins regulate such processes as translation and photosynthesis (Sugliani et al., 2016) as well as influence the accumulation of metabolites (Honoki et al., 2018) level of lipids (Maekawa et al., 2015) and phytohormones (Abdelkefi et al., 2018). They play the key role in plant development and growth, particularly during processes such as seed production (Masuda et al., 2008) and plant ageing (Sugliani et al., 2016). Plant RSHs are bifunctional proteins involving both the hydrolase domain (HD) and synthetase domain (SYNTH) of alarmones in their structure. The EF-hand motif found in CRSH responsible for Ca²⁺ ions binding belongs to other conserved domains within these proteins sequences (Tozawa et al., 2007, Turkan et al., 2023), together with ACT and TGS domains whose activity is probably connected with the recognition and binding of ligands (Shin et al., 2021). On the N-terminus of the amino acid chain of the RSH plant proteins there is also a sequence directing towards chloroplast (Boniecka et al., 2017). In the rape genome, 12 RSH genes which belong to 4 types: 6 *RSH1*, 2 *RSH2,* 3 *RSH3,* and 1 *CRSH*, located on 9 chromosomes are encoded, and particular isoforms most probably fulfil specific functions in the regulation of (pp)pGpp metabolism. Moreover, an *in silico* analysis of promoter regions of RSH genes in rape showed the presence of cis-regulatory responses elements to biotic factors (D 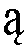 browska et al., 2021).

For analytical purposes, ppGpp is produced in bacterial systems. The level of these metabolites is strongly dependent on external factors and significantly elevated under stressful conditions. The reported maximum content of alarmones reached in bacteria was in the amount of ca. 470 nmol/g FW (Glazyrina et al., 2010; Traxler et al., 2008). The production of those substances in not limited to the bacteria kingdom, their accumulation is also observed in plants (unmodified *Arabidopsis arenosa* can produce about 50 pmol/g FW during a 6-day nitrogen deficit, 60 pmol/g FW, and in transgenic lines it can reach up to 600 pmol/g FW (Sugliani et al., 2016)) and animals (600 pmol/g FW in fruit fly pupas, and in transgenic flies - even 1200 times more (Ito et al., 2020), 40 pmol/g FW in human HeLa cells (Ito et al., 2020). Despite the high efficiency calculated per gram of fresh biomass, the ppGpp production in bacteria cannot be directly compared to those in other organisms since collecting 1 gram of bacterial sediment from a bacterial culture is far more difficult than growing 1 gram of tissue. It has also been observed that ppGpp visibly inhibits the growth of bacteria, which additionally hinders its simultaneous synthesis and biomass growth. In the case of another organism, i.e. the fruit fly, mass production of ppGpp would be exceptionally difficult. Efficient and large-scale fruit flies growing would be particularly problematic, especially because the mentioned transgenic flies do not survive when such a ppGpp accumulation level is reached. Additionally, for this purpose, consents of bioethics committees are required, especially in the case of transgenic organisms whose use in the European Union countries is significantly restricted.

The *Trichoderma* fungi settle virtually every latitude and climate zone (Hoyos-Carvajal et al., 2009a). Their spores can be separated from air, water reservoirs, soils, animals, and other fungi (Zeilinger et al., 2016), so they constitute an easily accessible research material. The growth of *Trichoderma* species is faster when compared to those of other fungi as the former take advantage of virtually all the food sources available, including the ability of growing on fruits, remains of dead matter, wood, polymer materials (Chen and Zhuang 2017; D 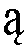 browska et al., 2021). They reproduce through agamogenesis and constitute the so-called anamorphia of the *Hypocrea* species, the sexually reproducing stage (Hermosa et al., 2012). When maturing, they develop spores on branched conidiophores (Schuster and Schmoll 2010) which can be colored green, yellow, or white. The fungi are characterized by high adaptability to various environmental conditions and different ways of living. *Trichoderma* are mainly antagonistic and mycoparasitic organisms also belonging to symbionts, i.e. microorganisms whose activity supports the growth of other organisms (Kubicek et al., 2011, Atanasova et al., 2013, Holzlechner et al., 2016). Moreover, certain *Trichoderma* species are opportunist, non-virulent symbionts of plants present in their rhizosphere, and they are known for their plant diseases fighting potential (Harman et al., 2004, Kredics et al., 2014).

The aim of the invention is to develop an efficient method of obtaining alarmones (ppGpp) from the *Trichoderma viride* fungus culture by extraction with the use of formic acid and ammonium acetate and its purification based on weak anions exchange in order to potentially implement these molecules in agriculture for the protection of crops against adverse stress factors, e.g. drought, pathogens.

The subject of the invention is the method of obtaining and extraction of secondary fungal metabolites characterized in that the *T. Viride* mycelium in the amount 75-100 mg is shredded and nucleotides are separated in such a manner that the mycelium is suspended in 1.5-2 N formic acid and incubated on ice preferably for 15 to 30 min, then centrifuged, and the supernatant is collected, and then ammonium acetate NH4OAc, the 13C ppGpp internal standard are added and introduced into an already prepared SPE column by rinsing with methanol MeOH and ammonium acetate in the amount from 35 to 60 mM, and then the column with the introduced sample is rinsed with ammonium acetate NH4OAc in the amount from 30 to 70 mM preferably 50 mM at the pH from 4 to 5 preferably pH 4.5 and methanol MeOH and nucleotides are eluted with a MeOH/ H2O/NH4OH solution, and the thus prepared eluate is lyophilized, dissolved in water and filtered in columns for DNA purification. Preferably the mycelium is shredded by grinding in a mortar preferably at the temperature from 20 to 25°C. Preferably centrifugation is performed under conditions 5000 x g for 5 to 15 min at the temperature from 0 to 8°C. Preferably after collecting the supernatant, ammonium acetate NH4OAc is added in the amount from 40 to 60 preferably 50 mM. Preferably it is rinsed with methanol in the amount from 10 to 80 mM. Preferably the MeOH/H2O/NH4OH solution is in the ratio from 10:60:5 to 30:80:20 preferably 20:70: 10 v/v/v. Preferably the lyophilizate is dissolved in water in the amount from 35 to 75 µl of water.

The method is effective and allows obtaining atypical nucleotides, alarmones, i.e. ppGpp from the fungi of the *Trichoderma* species in a cost-efficient manner. In a liquid culture under laboratory conditions, with the photoperiod as the inducing factor (in the range from 6 to 8 hours of light / from 18 to 16 of darkness), the obtained amount of ppGpp in the *T. viride* mycelium was only 10 times lower than in bacteria while at the same time high biomass was very easy to obtain. The *T. viride* fungi are easy to cultivate, and their proliferation for industrial purposes was optimized (e.g. (Nathan et al., 2014; Triveni et al., 2012)).

The method according to the invention has been revealed in the examples of preparation, whereby the examples do not exhaust all possibilities according to the method. The invention covers the whole range indicated in the patent claims.

### Example I.

### Materials and methods

### Cultivation of fungi

The *Trichoderma viride* strain was cultivated in a growth chamber at the temperature of 25°C and in constant darkness on the potato dextrose agar (PDA; Biomaxima, Lublin, Poland). Discs of mycelium were cut with a cork borer (the diameter of 1.0 cm corresponding to ca 4.0 mg of fresh biomass of fungi) and transferred from a PDA plate (potato extract (200g) (4.00), glucose (20.00), agar (15,00), the final pH at 25° C: 5.6 ± 0.2). After seven days of cultivation the newly grown mycelium of 1.0 cm in diameter was transferred on the potato dextrose broth (PDB; Biomaxima, Lublin, Poland; composition in g/l: potato extract (6,50), glucose (20,00), the final pH at 25°C: 5.6 ± 0.2) and further cultivated at the temperature of 25°C under the photoperiod conditions in the range from 6 to 8 h of light / from 18 to 16 h of darkness). After 7 days of cultivation, the *T. viride* mycelium was collected to test tubes of 5 ml volume and stored at the temperature of -80°C.

### Method of separation of alarmones from Trichoderma viride mycelium

The *T. viride* mycelium in the range from 75 to 100 mg was shredded by grinding in a mortar under conditions at the temperature range from 20 to 25°C. A nucleotide pool was separated from the *T. viride* fungus species according to a modified method described in papers (Ihara et al., 2015 and Bartoli et al., 2020).

The mycelium was suspended in 1.5-2 N formic acid (Sigma-Aldrich, St. Louis, USA) and incubated on ice for 15-30 min. Remains of cells were removed by centrifugation at 5000 x g for 5 to 15 min at the temperature range from 0 to 8°C and the supernatant was collected. Then ammonium acetate NH₄OAc (Sigma-Aldrich, St. Louis, USA) in the equal amount of 50 mM was added. The 13C ppGpp internal standard was added to each sample (the stage is omitted in the case when the amount of the product will not be further determined using the LC-MS/MS method) and the extract was introduced into an already prepared SPE column (Oasis WAX, Waters Corporation, Milford, USA) by rinsing with methanol (MeOH) (Sigma-Aldrich, Darmstadt, Germany) and 35-60 mM ammonium acetate. Then the columns with the introduced sample were rinsed with 50 mM NH₄OAc (pH 4.5) and MeOH. The nucleotide pool was eluted with the use of the MeOH/H₂O/NH₄OH solution mixed in the ratio (20:70:10 v/v/v). The eluate was lyophilized with the CentriVap Cold Trap system (Labconco Corporation, Kansas City, USA), dissolved in 60 of water in the range from 35 to 75 µl and filtered through the DNA purification columns (EurX, Gdańsk, Polska).

### Method of the quantitative determination of alarmones from the Trichoderma viride mycelium

The ppGpp content in extracts was determined with the use of liquid chromatography with the tandem mass spectrometry (UHPLC-ESI-MS/MS) using an integrated Nexera UHPLC and LCMS-8045 system (Shimadzu Corporation, Canby, OR, USA). We applied an Ascentis^{®} Express C18, 2,7 µm column (Supelco, Sigma-Aldrich, St. Louis, USA). The nucleotides were separated at the flow rate of 0.2-0.5 ml min⁻¹ in linear gradients of the solvent A which was the aqueous 8 mM hexylamine solution (Sigma-Aldrich, St. Louis, USA ), the pH 7.0-9.5 was adjusted with the use of acetic acid (Sigma-Aldrich, St. Louis, USA) as well as acetonitrile (Sigma-Aldrich, St. Louis, USA) as the solvent B set according to the following profile: 0 min, 96% A; 8 min, gradual change to 30% A, 8.50 min, change to 96% A; 20 min, 96% A. The source of the ESI ions was set on the positive ions mode, and the measurement parameters were automatically optimized. The endogenous internal standards 12C ppGpp and 13C ppGpp were detected in the multiple reactions monitoring mode (MRM). In order to detect 12C ppGpp ion transition was applied at m/z 603.8 → 152.1 and to detect 13C ppGpp m/z 613.9 → 157.05 .

### Results

### The evaluation of the fungus growth under different photoperiod conditions

The *T. viride* cultivation on a liquid medium was optimized. The highest fresh fungus biomass efficiency was obtained after 5- and 7- day cultivation on the PDA medium. It was shown that the mycelium phenotype changed depending on the photoperiod conditions. (Fig. 1).

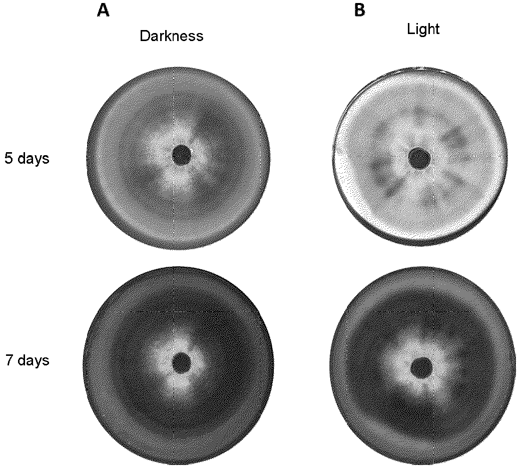
A
Darkness
B
Light
5 days
7 days

Fig. 1 The growth of the fungus on the solid PDA medium in darkness (A) and light (B) on the 5th and 7th day of *T. viride* growth

Moreover, it was shown that the photoperiod conditions not only caused a change in the fungus phototype but also influenced fresh *T. viride* biomass which was slightly higher in darkness than in the presence of light (Tab. 1).

**Tab. 1 Biomass of the T. viride mycelium obtained from the liquid cultivation on the liquid PDA medium dependent on the cultivation duration under light conditions, for n=3. The values are the mean of the results ± SD (n = 3).**

| **Time of fungus cultivation** | **Biomass of *T. viride* mycelium (g)** | |
|---|---|---|
| | **darkness** | **light** |
| 5 days | **10.92±0.02** | **10.65±0.05** |
| 7 days | **11.15±0.02** | **10.77±0.08** |

Slight differences in the *T. viride* growth rate were demonstrated for the fungus growing in darkness and in light. The results are however statistically insignificant. Obtaining the same amount of biomass of bacteria would require the use of a significantly higher amount of broth and providing the conditions of nutrient deficiency, while the method we offer requires only exposure of the culture to light of moderate intensity.

### Identification of alarmones in T. Viride biomass

The extraction of alarmones from the *T. viride* mycelium was performed and the content of those atypical nucleotides was determined in the biomass of the *T. viride* growing in light and in darkness with the use of liquid chromatography with tandem mass spectrometry (Fig. 2).

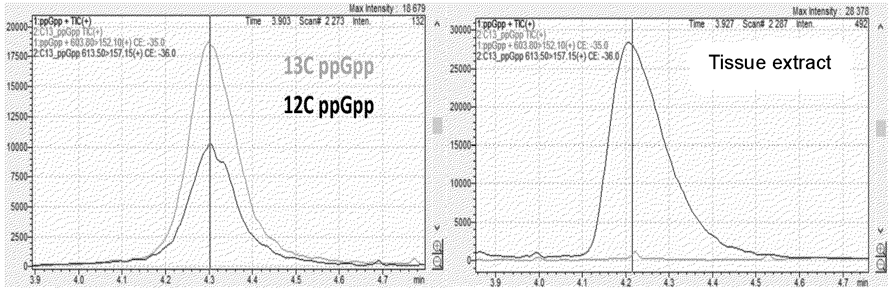

Fig. 2 Identification of ppGpp in extracts collected from the *T. Viride* mycelium.The result was obtained with the use of liquid chromatography with tandem mass spectrometry (LC-MS/MS) and the application of the integrated Nexera UHPLC and LCMS-8045 system. The chromatogram on the left side results from the analysis of the ppGpp nucleotides standard which is a mixture of isotopes of this compound: 12C, which occurs in tissues (black) and 13C, which does not naturally occur in tissues (magenta). The chromatogram on the right side results from the analysis of an ppGpp extract obtained from the mycelium. A high specificity of the analysis is based on a simultaneous maintenance of the both parameters characteristic of a given compound: the retention time which is the time required for a compound to pass through and leave the chromatographic column as the analysis proceeds as well as ion transition - the manner in which a given substance decomposes into smaller ions during spraying in the ESI part of the system.

The chromatographically determined amounts of alarmones were shown in Fig. 3 and Tab. 2. The obtained ppGpp efficiency from the *T. viride* mycelium amounted to 47 263 pmol/g in fresh biomass at the inducing factor (6-8 hours of light and 18-16 hours of darkness) for the synthesis of the compound (Tab. 2 and Fig. 3).

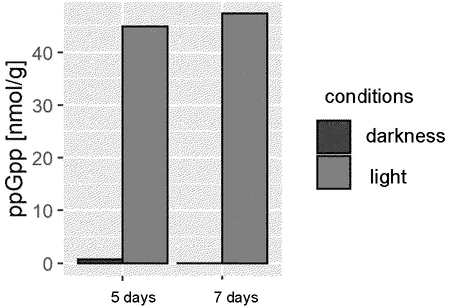

Fig. 3 The amount of ppGpp obtained from the extraction of the *T. viride* mycelium growing under different photoperiod conditions: control conditions (darkness) and the presence of the inducing factor (daylight).

**Tab. 2 The amount of alarmones obtained from the T. viride mycelium after the 5th and 7th day of cultivation in darkness or in the presence of light.**

| **Time of cultivation** | **Amount of ppGpp [nmol/g]** | |
|---|---|---|
| | **darkness** | **light** |
| 5 days | 0.72 | 44.82 |
| 7 days | 0.00 | 47.26 |

### Example II.

The method of obtaining and extraction of secondary fungal metabolites is characterized in that the *T. Viride* mycelium in the amount of 75 mg is shredded and nucleotides are separated in such a manner that the mycelium is suspended in 1.5 N formic acid and incubated on ice preferably for 15 min, then centrifuged, and the supernatant is collected, and then ammonium acetate NH4OAc, the 13C ppGpp internal standard are added and introduced into an already prepared SPE column by rinsing with methanol MeOH and ammonium acetate in the amount of 35 mM, and then the column with the introduced sample is rinsed with ammonium acetate NH4OAc in the amount of 30 mM at the pH 4 and methanol MeOH and nucleotides are eluted with a MeOH/H2O/NH4OH solution, and the thus prepared eluate is lyophilized, then dissolved in water and filtered in columns for DNA purification.

### Example III

The method of obtaining and extraction of secondary fungal metabolites is characterized in that the *T. Viride* mycelium in the amount of 100 mg is shredded and nucleotides are separated in such a manner that the mycelium is suspended in 2 N formic acid and incubated on ice for 30 min, then centrifuged, and the supernatant is collected, and then ammonium acetate NH4OAc, the 13C ppGpp internal standard are added and introduced into an already prepared SPE column by rinsing with methanol MeOH and ammonium acetate in the amount of 60 mM, and then the column with the introduced sample is rinsed with ammonium acetate NH4OAc in the amount of 70 mM, at the pH 4 and methanol MeOH and nucleotides are eluted with a MeOH/H2O/NH4OH solution, and the thus prepared eluate is lyophilized, dissolved in water and filtered in columns for DNA purification. The mycelium is shredded by grinding in a mortar preferably at the temperature of 20°C. Centrifugation is performed under conditions 5000 x g for 10min at the temperature of 0°C. After collecting the supernatant, ammonium acetate NH4OAc is added in the amount of 50 mM. It is rinsed with methanol in the amount of 50 mM. The MeOH/H2O/NH4OH solution is in the ratio 20:70:10 v/v/v. The lyophilizate is dissolved in the amount of 35 µl of water.

### Example IV

The method of obtaining and extraction of secondary fungal metabolites is characterized in that the *T. Viride* mycelium in the amount of 85 mg is shredded and nucleotides are separated in such a manner that the mycelium is suspended in 1.75 N formic acid and incubated on ice for 20 min, then centrifuged, and the supernatant is collected, and then ammonium acetate NH4OAc, the 13C ppGpp internal standard are added and introduced into an already prepared SPE column by rinsing with methanol MeOH and ammonium acetate in the amount of 40 mM, and then the column with the introduced sample is rinsed with ammonium acetate NH4OAc in the amount of 45 mM, at the pH 4 and methanol MeOH and nucleotides are eluted with a MeOH/H2O/NH4OH solution, and the thus prepared eluate is lyophilized, then dissolved in water and filtered in columns for DNA purification. The mycelium is shredded by grinding in a mortar preferably at the temperature of 25°C. Centrifugation is performed under conditions 5000 x g for 5 min at the temperature of 8°C. After collecting the supernatant, ammonium acetate NH4OAc is added in the amount of 40 mM. It is rinsed with methanol in the amount of 80 mM. The MeOH/H2O/NH4OH solution is in the ratio 10:60:5 v/v/v. The lyophilizate is dissolved in water in the amount of 75 µl of water.

## Claims

1. The method of obtaining and extraction of secondary fungal metabolites **characterized in that** the *T. Viride* mycelium in the amount 75-100 mg is shredded and nucleotides are separated in such a manner that the mycelium is suspended in 1.5-2 N formic acid and incubated on ice preferably for 15 to 30 min, then centrifuged and the supernatant is collected, and then ammonium acetate NH₄OAc, the 13C ppGpp internal standard are added and introduced into an already prepared SPE column by rinsing with methanol MeOH and ammonium acetate in the amount from 35 to 60 mM, and then the column with the introduced sample is rinsed with ammonium acetate NH₄OAc in the amount from 30 to 70 mM preferably 50 mM at the pH from 4 to 5 preferably pH 4.5 and methanol MeOH and nucleotides are eluted with a MeOH/H₂O/NH₄OH solution, and the thus prepared eluate is lyophilized, dissolved in water and filtered in columns for DNA purification.

2. Method according to claim 1 **characterized in that** the mycelium is shredded by grinding in a mortar preferably at the temperature from 20 to 25°C.

3. Method according to claims 1 or 2 **characterized in that** centrifugation is performed under conditions 5000 x g for 5 to 15 minutes at the temperature from 0 to 8°C.

4. Method according to claims 1, 2, or 3 **characterized in that** after collecting the supernatant, ammonium acetate NH₄OAc is added in the amount from 40 to 60 mM preferably 50 mM.

5. Method according to claims 1, 2, 3, or 4 **characterized in that** it is rinsed with methanol in the amount from 10 to 80 mM.

6. Method according to claims 1, 2, 3, 4, or 5 **characterized in that** the MeOH/H₂O/ NH₄OH solution is in the ratio from 10:60:5 to 30:80:20 preferably 20:70:10 v/v/v.

7. Method according to claims 1, 2, 3, 4, 5, or 6 **characterized in that** the lyophilizate is dissolved in water in the amount from 35 to 75 µl of water.
